(19) **Europäisches Patentamt**
**European Patent Office**
**Office européen des brevets**

(11) **EP 4 514 268 B1**

(12) **EUROPÄISCHE PATENTSCHRIFT**

(45) Veröffentlichungstag und Bekanntmachung des Hinweises auf die Patenterteilung:
**25.02.2026 Patentblatt 2026/09**

(21) Anmeldenummer: **23723435.6**

(22) Anmeldetag: **26.04.2023**

(51) Internationale Patentklassifikation (IPC):
**A61C 13/08** *(2006.01)* **A61C 19/10** *(2006.01)*
**A61C 13/083** *(2006.01)* **A61C 13/09** *(2006.01)*

(52) Gemeinsame Patentklassifikation (CPC):
**A61C 13/082; A61C 13/081; A61C 13/083; A61C 13/09; A61C 19/10**

(86) Internationale Anmeldenummer:
**PCT/EP2023/060909**

(87) Internationale Veröffentlichungsnummer:
**WO 2023/208991 (02.11.2023 Gazette 2023/44)**

(54) **VERFAHREN ZUR HERSTELLUNG EINER ZAHNRESTAURIERUNG MIT EINEM ZIELFARBWERT, ENTSPRECHENDES KIT UND VERWENDUNG EINER GLASUR IN DEM VERFAHREN.**

METHOD FOR PRODUCING A DENTAL RESTORATION HAVING A TARGET COLOR VALUE, CORRESPONDING KIT AND USE OF GLAZING IN THE METHOD.

PROCÉDÉ DE RÉALISATION D'UNE RESTAURATION DENTAIRE AYANT UNE VALEUR DE COULEUR CIBLE, KIT CORRESPONDANT ET UTILISATION DU GLAÇAGE DANS LE PROCÉDÉ.

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **27.04.2022 EP 22170330**

(43) Veröffentlichungstag der Anmeldung:
**05.03.2025 Patentblatt 2025/10**

(73) Patentinhaber: **Vita Zahnfabrik H. Rauter GmbH & Co. KG**
**79713 Bad Säckingen (DE)**

(72) Erfinder:
• **GOEDIKER, Michael**
**79713 Bad Säckingen (DE)**

• **GOEDIKER, Berit**
**79713 Bad Säckingen (DE)**

(74) Vertreter: **dompatent**
**Partnerschaft von Patentanwälten und Rechtsanwälten mbB**
**Deichmannhaus am Dom**
**Bahnhofsvorplatz 1**
**50667 Köln (DE)**

(56) Entgegenhaltungen:
WO-A1-2020/185086   CN-A- 108 784 875
DE-A1- 102015 204 109   US-A- 4 828 117
US-A- 5 906 490   US-A1- 2020 330 331
US-A1- 2021 322 283

**Beschreibung**

Die vorliegende Erfindung betrifft ein Verfahren gemäß Anspruch 1 zur Herstellung einer dentalen Restauration, wobei die Farbgebung durch Glasieren eines Formkörpers mit einer farbigen Glasur eines definierten Farbwerts und Wärmebehandeln des glasierten Formkörpers erreicht wird. Auch Teil der Erfindung sind ein Kit gemäß Anspruch 10 und die Verwendung einer Glasur gemäß Anspruch 14.

Der Begriff der restaurativen Zahnmedizin bezeichnet die Behandlungen von Zahnproblemen bei gleichzeitig funktioneller und ästhetischer Wiederherstellung der Zähne. Dabei steht insbesondere das naturgetreue Nachempfinden der Zähne im Vordergrund, das in vielen Fällen über die ästhetische Akzeptanz einer dentalen Restauration entscheidet. Zu diesem Zweck stehen in der Regel eine Reihe von Grundfarben zur Verfügung, die farblich an die bestehende Zahnsituation angepasst werden.

Die konventionelle Farbbestimmung von Zähnen erfolgt in der Regel mit Hilfe sogenannter Farbringe oder -skalen, die mit Farbmustern versehen sind und zum visuellen Farbabgleich dienen, wobei die Zusammenstellung der Farbmuster traditionell den am häufigsten in der Natur vorkommenden Zahnfarben, welche empirisch ermittelt wurden, entspricht.

In diesem Zusammenhang beschreibt DE 693 19 291 eine Farbmischungs-Indikatorvorrichtung, die bei der Auswahl von gewünschten künstlichen Zahnfarben bei der Herstellung von Brücken, Kronen, Teilgebissen und dergleichen Anwendung findet. Offenbart wird ein Set von Zahnfarbenmischungs-Indikatorvorrichtungen, wobei jede beteiligte Indikatorvorrichtung eine Mehrzahl von Farbmustern aufweist, die in einer Anordnung vorgesehen sind und um ein zentrales Muster der Farbmuster angeordnet sind, wobei jedes Muster von diesen benachbarten Mustern beabstandet ist, wobei die Farben der Muster Farben eines vorbestimmten Farbsystems darstellen, das ein dreidimensionales Koordinatensystem (L, a, b) aufweist, das mit der genannten Anordnung ausgewichtet ist, wobei die Farbe jedes Musters der Farbe an der entsprechenden Position in dem Koordinatensystem (L, a, b) entspricht, wobei die Farben der Muster jeder beteiligten Vorrichtung in einer Ebene des Koordinatensystems liegen und die jeder Vorrichtung entsprechende Ebene im Wesentlichen parallel zu und im Abstand von den Ebenen der anderen beteiligte Vorrichtungen angeordnet ist, und wobei im Gebrauch des Sets ein Zahn eines Patienten mit jeglicher der beteiligten Vorrichtungen verglichen werden kann und eine gewünschte Zahnfarbe zwischen einem ausgewählten Paar von Farbmustern zur Herstellung gemischt werden kann, indem eine Mischung der Farbmaterialien gebildet wird, die zur Herstellung des ausgewählten Paares verwendet werden, wobei die zentralen Farbmuster einander benachbarter Ebenen entlang einer gemeinsamen Linie angeordnet sind und in Bezug auf eine Achse (L) des Koordinatensystems versetzt sind, die zu einander benachbarter Ebenen senkrecht sind.

Mit Hilfe solcher Farbskalen erfolgt eine schrittweise Bestimmung der Zahngrundfarbe gemäß den drei Farbdimensionen Helligkeit, Farbintensität und Farbton, die den menschlichen Farbeindruck bestimmen. Entsprechende Farbskalen sind beispielsweise unter der Bezeichnung VITA SYSTEM 3D-MASTER® (vgl. Figur 2) kommerziell erhältlich.

Um eine objektivere Erfassung der Zahnfarbe zu erzielen, schlägt der Stand der Technik eine Reihe von technisch unterstützten Verfahren vor.

EP 3 643 272 beschreibt ein Verfahren zur Festlegung einer Zahnfarbe einer Füllung oder sonstigen Restauration, die insbesondere unter Verwendung von Composite-Materialien hergestellt wird, wobei mindestens ein Zahn hinsichtlich seines Farbwerts elektronisch erfasst, insbesondere gescannt wird, wobei natürliche Zähne vorab spektral gemessen werden, wobei die gemessenen Farbwerte in einem Farbraum in mehrere, insbesondere vier, Kategorien, die in dem Farbraum je eine dreidimensionale Farbwolke bilden, eingeteilt werden, und zwar mittels eines strukturerkennenden Algorithmus, und wobei die Zahnfarbe der Füllung festgelegt wird, indem ermittelt wird, zu welcher der Kategorien der Zahn den geringsten Farbabstand hat oder in welche Farbwolke der Zahn fällt.

WO 2020/156855 offenbart ein Unterstützungssystem zur Zahnbehandlung, insbesondere durch Veränderung der Zahnfarbe, mit einem Messgerät, welches die Zahndaten zu behandelnder Zähne, insbesondere die Zahnfarbe sowie Zahnkoordinaten, bestimmt, einer Datenverarbeitungseinrichtung sowie einer Kamera zur Aufnahme eines Zahnbilds, wobei die Datenverarbeitungseinrichtung vorzugsweise ein Empfangsmodul und das Messgerät ein Sendemodul zur Übertragung gemessener Zahndaten vom Messgerät an die Datenverarbeitungseinrichtung aufweist, einem in die Datenverarbeitungseinrichtung integrierten Zuordnungsmodul zur Zuordnung der empfangenen Zahndaten zu einem Zahnbild, einer Auswahleinrichtung zum Auswählen eines Farbveränderungsgrades und einer Anpassungsvorrichtung zur Anpassung des Zahnbilds an den ausgewählten Farbveränderungsgrad.

EP 3 650 821 betrifft ein System zur Bestimmung der Farbe von Zähnen, das eine 2D- oder 3D-Kamera umfasst, wobei das System angepasst ist, um Bilder zu erfassen, die durch die Kamera aufgenommen werden, und einen Aufsatz, wobei der Aufsatz einen Abstandshalter umfasst, der eine Verbindungeinrichtung zum Anbringen des Aufsatzes an der Kamera aufweist, wobei der Abstandshalter eine Blende umfasst, mittels der die Kamera angepasst ist, um ein Bild von Zähnen aufzunehmen, wobei der Abstandshalter angepasst ist, um einen Abstand zwischen einer Aufnahmevorrichtung der Kamera und der Zahnoberfläche zu erzeugen, und wobei der Abstandshalter angepasst ist, um einen Abstand zwischen der Aufnahmevorrichtung der Kamera und der Blende zu erzeugen, wobei die Kamera einen normalen Aufzeichnungsmodus ohne die Verwendung des Aufsatzes und einen Farbmessmodus zur Verwendung mit einem Aufsatz zum Messen der Farbe des Zahns aufweist, wobei das System zum Erkennen des Vorhandenseins des Aufsatzes auf der Kamera auf der Basis der Änderungen in dem durch die Kamera aufgenommenen Bildfeld angepasst ist, um in einen Farbmessmodus umzuschalten, wenn diese Änderungen im Bildfeld detektiert werden.

WO 2020/154450 bezeichnet ein Verfahren zur Farbanpassung, das die folgenden Schritte umfasst: Bereitstellen von Zahnmessdaten, wobei die Zahnmessdaten mindestens einen Einsatzbereich umfassen, in den die Zahnrestauration eingesetzt werden soll; Bereitstellen von Restaurationsdaten; Bereitstellen von Informationen über Färbe- und Glasurfarben; Erzeugen einer Farbcharakteristik aus den Zahnmessdaten oder anderen Informationen unter Berücksichtigung der Restaurationsdaten und Berechnen einer mehrfarbigen Zielfärbung aus der Farbcharakteristik unter Berücksichtigung von Informationen über Färbe- oder Glasurfarben, und Ausgeben der mehrfarbigen Zielfärbung zur Farbanpassung der Zahnrestauration.

DE 10 2015 204 109 beschreibt eine sprühbare Verblendkeramik-Zusammensetzung für dentale Restaurationen aus Zirkoniumdioxid umfassend eine disperse Phase und ein Dispersionsmedium, wobei die disperse Phase eine pulverisierte Glaskeramik, umfassend $SiO_2$, $Nb_2O_5$, $Al_2O_3$, $Li_2O$, $Na_2O$ und $ZrO_2$, und das Dispersionsmedium Isopropanol und/oder Ethanol und/oder Ethylenglykol enthält und wobei die sprühbare Verblendkeramik-Zusammensetzung mindestens ein Treibmittel enthält. Um die Ästhetik der Verblendkeramik zu optimieren, kann die pulverisierte Glaskeramik als färbende oder fluoreszierende Zusätze Pigmente enthalten.

US 2021/0322283 offenbart eine Glasur-Zusammensetzung für dentale Restaurationen aus Zirkoniumoxid. Die Glasur-Zusammensetzung umfasst eine Flüssigkeit, Glas-Partikel und hydrophile Silica-Nanopartikel.

US 4,828,117 beschreibt eine mehrschichtige dentale Restauration mit einer transluzenten Porzellanschicht, die eine opakere keramische Schicht bedeckt, wobei die Farbe der beiden Schichten aufeinander abgestimmt sind.

CN 108784875 betrifft ein Farbkartenmuster aus Zirkoniumoxid.

WO 2020/185086 bezieht sich auf ein Verfahren, mit dem einer dentalen Restauration eine natürliche Farbe und optische Tiefe verliehen werden soll, wobei sich die Farbe und optischen Eigenschaften an denen eines benachbarten oder angrenzenden Zahns orientieren.

US 5,906,490 stellt ein Kit und ein Verfahren für die Herstellung von dentalen Produkten bereit, wobei drei Materialien mit unterschiedlichen Helligkeitswerten jeweils zu Schichten mit zunehmender Opazität geformt werden.

Sowohl die konventionelle, also die subjektiv-visuelle Zahnfarbenstimmung, als auch die technisch unterstützte objektivierte Zahnfarbmessung werden durch viele Einflussfaktoren bestimmt, so dass das ästhetische Ergebnis oft von dem Können und der Erfahrung des Zahnarztes und des Zahntechnikers abhängt. Weiterhin weist das bestehende System den Nachteil auf, dass eine große Anzahl von Proben vorgehalten werden muss, um eine möglichst farbgetreue Anpassung zu erzielen. In den Fällen, in denen eine falsche Wahl getroffen wurde, müssen außerdem aufwendige Farbanpassungen und -korrekturen vorgenommen werden.

Insofern besteht weiterhin der Bedarf nach einem vereinfachten und verlässlichen System zur Auswahl und Herstellung dentaler Restaurationen mit einer natürlichen Ästhetik, die sich in das bestehende Zahnschema einpassen. Darüber hinaus ist es wünschenswert, eine einfache Anpassung der Zahnfarbe vornehmen zu können.

Vor diesem Hintergrund stellt die vorliegende Erfindung ein Verfahren zur Herstellung einer dentalen Restauration bereit, das eine einfache und effiziente Farbanpassung erlaubt und durch das die Anzahl an Proben, die für eine genaue Farbanpassung vorgehalten werden muss, deutlich reduziert werden kann.

Ein erster Gegenstand der vorliegenden Erfindung ist daher ein Verfahren zur Herstellung einer dentalen Restauration, umfassend die folgenden Schritte:

a) Bereitstellen eines Formkörpers für dentale Restaurationen, wobei der Formkörper einen Grundfarbwert FW(F) aufweist und wobei der Grundfarbwert FW(F) des Formkörpers bezüglich eines Zielfarbwerts FW(Z) der dentalen Restauration einen maximalen Differenzwert AE von 5 aufweist;
b) Aufbringen einer Glasur auf den Formkörper, wobei die Glasur einen definierten Farbwert FW(Gx) aufweist;
c) Wärmebehandeln des glasierten Formkörpers unter Erhalt der dentalen Restauration,

dadurch gekennzeichnet, dass der definierte Farbwert FW(Gx) der Glasur der Differenz AE zwischen den Farbwerten FW(Z) und FW(F) entspricht, wobei es sich bei den Farbwerten um CIEL*a*b*-Werte handelt und die und die Glasur in einer Dicke von 10 bis 50 µm, vorzugsweise 20 bis 40 µm auf den Formkörper aufgetragen wird.

Bei den Farbwerten und Differenzwerten handelt es sich um CIEL*a*b*-Werte, wobei sich die Differenz vorzugsweise auf jeweils einen der Werte bezieht. Die Bestimmung solcher Werte ist dem Fachmann bekannt und beispielsweise von A. Baltzer und V. Kaufmann-Jinoian in "Die Bestimmung der Zahnfarben", erschienen in QZ Quintessenz Zahntechnik, 30. Jahrgang, Juli 2004, beschrieben.

Während herkömmliche Verfahren zur Herstellung dentaler Restaurationen auf die Erfahrung des Zahntechnikers vertrauen und die Farbanpassung der Restauration meist manuell erfolgt, setzt das erfindungsgemäße Verfahren auf eine gezielte Auswahl von Glasuren mit einem definiertem Farbwert, der der Differenz zwischen Grundfarbwert und Zielfarbwert des Formkörpers entspricht, so dass das empirische Vorgehen, das dem herkömmlichen Verfahren zugrunde liegt, entfällt. Vielmehr wird durch gezielte Auswahl einer Glasur geeigneter Farbe eine gezielte Anpassung des Formkörpers ermöglicht. Daher wird in einer bevorzugten Ausführungsform die Glasur mit definiertem Farbwert $FW(G_x)$ durch Auswahl aus einer Reihe von Glasuren mit definierten Farbwerten $FW(G)$ bereitgestellt.

Die Glasur wird vorzugsweise derart ausgewählt, dass ihr Farbwert $FW(Gx)$ der Differenz zwischen Grundfarbwert $FW(F)$ des Formkörpers und Zielfarbwert $FW(Z)$ des Formkörpers entspricht. Daher gilt beispielhaft:

$$FW(Z_1) = FW(F_1) + FW(G_1)$$

Anders ausgedrückt gilt:

$$FW(G_x) = FW(Z_y) - FW(F_z) \text{ mit } y = x + z \text{ und } y\text{-}z < 5$$

In den Fällen, in denen keine passende Zahnfarbe zur Verfügung steht oder vom Anwender eine falsche Auswahl getroffen wurde, sind oftmals manuelle Farbkorrekturen notwendig. Dazu muss zunächst die richtige keramische Malfarbe mit Pinsel oder Spray an der richtigen Stelle aufgebracht werden, was eine gewisse Erfahrung voraussetzt. Darauf erfolgt ein sogenannter Malfarbenfixierbrand. Anschließend wird eine Glasur aufgetragen und ein Glasurbrand durchgeführt, erst dann kann die Farbwirkung definitiv beurteilt werden. Durch das erfindungsgemäße Verfahren kann die farbliche Anpassung mit nur einem Brand erfolgen, wodurch sich das Ergebnis schneller beurteilen lässt.

Anders als herkömmliche Verblendungen, die in der Regel farblos sind und zur Erzielung eines bestimmten Glanzes eingesetzt werden, verwendet das erfindungsgemäße Verfahren farbige Glasuren, wobei die Farbgebung vorzugsweise durch farbige Pigmente erfolgt. In einer bevorzugten Ausführungsform des erfindungsgemäßen Verfahrens werden daher Glasuren verwendet, die eine flüssige Anmischphase; und eine feste Phase umfassend färbende Substanzen, vorzugsweise farbige Pigmente, aufweisen, wobei der Anteil der flüssigen Anmischphase in der Glasur mindestens 60 Gew.-% beträgt, bezogen auf das Gesamtgewicht der Glasur.

Auf diese Weise kann eine Auswahl von Glasuren mit definiertem Farbwert bereitgestellt werden, aus der die Glasur ausgewählt wird, deren Farbwert der Differenz zwischen dem Grundfarbwert des Formkörpers und des zu erreichenden Zielfarbwerts entspricht. So entfällt zum einen der Schritt des empirischen Anmischens der Glasur und zum anderen wird der Zielfarbwert durch Auftragung nur einer Glasur erreicht.

Im Rahmen des erfindungsgemäßen Verfahrens erfolgt die Bereitstellung des Formkörpers vorzugsweise durch Auswahl aus einem Set von Formkörpern mit jeweils einem definierten Grundfarbwert $FW(F)$ durch Vergleich mit den zur Restauration benachbarten Zähnen im Mund des Patienten, vorzugsweise mit Hilfe eine Farbskala zur Zahnfarbenbestimmung. Durch diese Art der Bereitstellung in Kombination mit der farblichen Anpassung durch Auftragen einer ausgewählten Glasur ist es möglich, ein begrenztes Angebot an unterschiedlichen Grundfarben bereitzuhalten, deren Farbe durch eine entsprechende Glasur angepasst werden kann. Die Notwendigkeit ein komplettes Farbsortiment an Rohlingen vorzuhalten entfällt somit. Vielmehr besteht nun die Möglichkeit, mit Hilfe unterschiedlicher farblicher Glasuren ausgehend von definierten Ausgangspunkten bestimmte Farborte zu erreichen. Daher handelt es sich bei dem Grundfarbwert und dem Zielfarbwert vorzugsweise jeweils um definierte Farbwerte, wie sie in einer standardisierten Farbskala zur Zahnfarbenbestimmung festgelegt sind. Solche standardisierten Farbskalen sind dem Fachmann bekannt und beispielsweise unter der Bezeichnung VITA classical A1-D4®, VITA SYSTEM 3D-MASTER® oder VITA Toothguide 3D-MASTER® kommerziell erhältlich (vgl. hierzu auch Figur 2).

Um den Einfluss der subjektiven Wahrnehmung von Farben bei der Farbauswahl zu minimieren, hat es sich als vorteilhaft erwiesen, digitale Unterstützung heranzuziehen. Daher ist eine Ausführungsform des erfindungsgemäßen Verfahrens bevorzugt, in der die Bestimmung des $\Delta E$ und/oder die Auswahl des Formkörpers computergestützt erfolgt.

Gemäß dem erfindungsgemäßen Verfahren wird der Zielfarbwert $FW(Z)$ der dentalen Restauration durch Auftrag einer entsprechenden Glasur erreicht. Hierbei hat es sich als vorteilhaft erwiesen, die Dicke, in der die Glasur aufgetragen wird zu begrenzen, um so eine Beeinträchtigung des Farbergebnisses zu minimieren.

Laut der Erfindung wird die Glasur daher in einer Dicke von 10 bis 50 $\mu m$, vorzugsweise 20 bis 40 $\mu m$ auf den Formkörper aufgetragen. Das Auftragen der Glasur kann dabei mit dem Fachmann bekannten Verfahren manuell oder maschinell erfolgen.

Das erfindungsgemäße Verfahren verwendet Glasuren mit definierten Farbwerten, um eine Anpassung der Farbe eines bereitgestellten Formkörpers zu erreichen. Ein weiterer Gegenstand der vorliegenden Erfindung ist daher eine Glasur zur Verwendung in dem erfindungsgemäßen Verfahren, wobei die Glasur

    i) eine flüssige Anmischphase; und
    ii) eine feste Phase umfassend färbende Substanzen, vorzugsweise farbige Pigmente,

aufweist, wobei der Anteil der flüssigen Anmischphase in der Glasur mindestens 60 Gew.-% beträgt, bezogen auf das Gesamtgewicht der Glasur und wobei die Glasur einen definierten Farbwert $FW(G_x)$ aufweist.

Der definierte Farbwert $FW(G_x)$ der Glasur entspricht der Differenz $\Delta E$ zwischen dem Grundfarbwert $FW(F)$ eines Formkörpers und dem Zielfarbwert $FW(Z)$ einer aus diesem Formkörper herzustellenden dentalen Restauration.

Die Anmischphase ist vorzugsweise ausgewählt aus der Gruppe bestehend aus Wasser, einem oder mehreren Alkoholen und Mischungen hiervon. Der Alkohol ist vorzugsweise ausgewählt aus der Gruppe der 1-, 2- und 3-wertigen Alkohole sowie Mischungen hiervon. In einer besonders bevorzugten Ausführungsform ist der Alkohol ausgewählt aus der Gruppe bestehend aus Glycerin, 1,3-Butandiol, 1,2-Propandiol, Ethanol und Mischungen hiervon.

In einer bevorzugten Ausführungsform umfasst die Anmischphase weiterhin einen oder mehrere Komplexbildner, vorzugsweise ausgewählt aus der Gruppe bestehend aus Ethylendiamintetraessigsäure und Citronensäure.

Als Anmischphase können solche verwendet, die kommerziell erhältlich sind, wie beispielsweise Triplex® Lösung B.

Als feste Phase der Glasur können im Stand der Technik bekannte Zusammensetzungen verwendet werden. Vorzugsweise wird bei der Glasur als feste Phase auf eine Grundmischung zurückgegriffen, der je nach Bedarf färbende Substanzen, insbesondere farbige Pigmente, beigemischt werden. In einer bevorzugten Ausführungsform umfasst die feste Phase der Glasur 45 bis 70 Gew.-% $SiO_2$, besonders bevorzugt 50 bis 65 Gew.-%, bezogen auf das Gesamtgewicht der festen Phase. Weiterhin bevorzugt ist eine Ausführungsform, in der die feste Phase eine oder mehrere der folgenden Komponenten umfasst, jeweils bezogen auf das Gesamtgewicht der festen Phase:

- 5 bis 15 Gew.-%, vorzugsweise 7 bis 13 Gew.-% $Al_2O_3$;
- 5 bis 15 Gew.-%, vorzugsweise 7 bis 13 Gew.-% $B_2O_3$
- 5 bis 15 Gew.-%, vorzugsweise 7 bis 13 Gew.-% $K_2O$;
- 5 bis 15 Gew.-%, vorzugsweise 7 bis 13 Gew.-% $Na_2O$.

Um die gewünschten Farbwerte zu erreichen kann die feste Phase bis zu 5 Gew.-% an färbenden Substanzen, insbesondere farbige Pigmente, umfassen, bezogen auf das Gesamtgewicht der festen Phase.

Als färbende Substanzen können in der Branche übliche Pigmente und Pigmentmischungen eingesetzt werden, wie sie in der Keramik-, Glas- und Porzellanindustrie verwendet werden. Insofern weist die erfindungsgemäße Glasur eine hohe Kompatibilität mit bestehenden Systemen auf.

In einer weiterhin bevorzugten Ausführungsform liegt die Glasur vorzugsweise als Kit vor, umfassend einen Behälter mit der Anmischphase und einen Behälter mit der festen Phase umfassend färbende Substanzen. Diese Form der Bereitstellung bietet den Vorteil, dass die gewünschte Glasur vor Ort angerührt werden kann und einem Eintrocknen durch Lagerung vorgebeugt wird.

Die vorliegende Erfindung hat das Ziel, die Herstellung von dentalen Restaurationen mit gewünschten Farbwerten zu erleichtern. In diesem Zusammenhang ist ein weiterer Gegenstand der vorliegenden Erfindung ein Kit zur Herstellung einer dentalen Restauration umfassend

a) ein Sortiment an Glasuren für Formkörper für dentale Restaurationen, wobei jede Glasur einen definierten Farbwert $FW(G_x)$ aufweist, wobei die Farbwerte $FW(G_x)$ den Differenzen $\Delta E$ zwischen den Farbmustern einer standardisierten Farbskala zur Zahnfarbenbestimmung entsprechen und wobei $\Delta E$ nicht größer als 5 ist, und

b) einen Farbführer zur Zuordnung eines ermittelten $\Delta E$-Werts zu der entsprechenden Glasur.

Das erfindungsgemäße Kit findet vorzugsweise in dem erfindungsgemäßen Verfahren Anwendung und erlaubt es, ausgehend von einem Formkörper mit einem Grundfarbwert $FW(F)$ durch Auswahl einer geeigneten Glasur auf eine einfache Weise und mit nur einer Wärmebehandlung eine dentale Restauration mit einem Zielfarbwert $FW(Z)$ zu erreichen, wobei der Farbwert $FW(G)$ der Glasur der Differenz $\Delta E$ zwischen dem Grundfarbwert $FW(F)$ des Formkörpers und dem Zielfarbwert $FW(Z)$ der dentalen Restauration entspricht. Das erfindungsgemäße Kit ermöglicht durch die Bereitstellung eines Farbführers eine gezielte Auswahl der geeigneten Glasur, die der ermittelten Farbwertdifferenz $\Delta E$ zwischen Grundfarbwert $FW(F)$ und Zielfarbwert $FW(Z)$ entspricht. Durch Auftragen der Glasur und Wärmebehandeln kann somit der Farbunterschied ausgeglichen werden und eine dentale Restauration zur Verfügung gestellt werden, die sich ästhetisch in das bestehende Zahnschema des Patienten einfügt.

Dem Fachmann stehen eine Reihe von standardisierten Farbskalen zur Verfügung, die zur Zahnfarbenbestimmung eingesetzt werden. Vorzugsweise handelt es sich bei der standardisierten Farbskala, die Teil des erfindungsgemäßen Kits sein kann, um VITA classical A1-D4®, VITA SYSTEM 3D-MASTER® oder VITA Toothguide 3D-MASTER®.

In einer bevorzugten Ausführungsform umfasst das erfindungsgemäße Kit weiterhin eine standardisierte Farbskala zur Zahnfarbenbestimmung.

Das erfindungsgemäße Kit ermöglicht es, durch eine begrenzte Anzahl von Glasuren ein breites Farbspektrum zu erreichen. In einer bevorzugten Ausführungsform umfasst das Kit ein Sortiment von 2 oder mehr, vorzugsweise 3 oder mehr, besonders bevorzugt 4 oder mehr Glasuren und/oder nicht mehr als 10, vorzugsweise nicht mehr als 8, besonders bevorzugt nicht mehr als 6 Glasuren.

In einer weiterhin bevorzugten Ausführungsform umfasst das Kit weiterhin einen Applikator zum Aufbringen der Glasur auf einen Formkörper für dentale Restaurationen. In einer ebenfalls bevorzugten Ausführungsform umfasst das erfindungsgemäße Kit neben dem Sortiment an Glasuren, dem Farbführer und optional dem Applikator weiterhin ein Sortiment an Formkörpern für dentale Restaurationen mit jeweils einem Grundfarbwert $FW(F_x)$, die vorzugsweise den Farbwerten einer standardisierten Farbskala zur Zahnfarbenbestimmung entsprechen. Auf diese Weise kann ein Komplettpaket zur Verfügung gestellt werden, das eine einfache und effiziente Herstellung dentaler Restaurationen erlaubt.

Vorzugsweise handelt es sich bei den Glasuren des erfindungsgemäßen Kits um solche gemäß der vorliegenden Erfindung.

Ein weiterer Gegenstand der vorliegenden Erfindung ist die Verwendung einer Glasur mit einem definierten Farbwert $FW(G_x)$ in der Herstellung dentaler Restaurationen, wobei der Farbwert $FW(G_x)$ der Differenz $\Delta E$ zwischen den Farbmustern einer standardisierten Farbskala zur Zahnfarbenbestimmung entspricht, wobei $\Delta E$ nicht größer als 5 ist.

Figur 1 und das nachfolgende Beispiel verdeutlichen die vorliegende Erfindung, sind jedoch keineswegs als Einschränkung des Erfindungsgedanken zu verstehen.

Figur 1 verdeutlicht das erfindungsgemäß Konzept, bei dem ausgehend von dentaler Restauration, wie sie beispielsweise kommerziell erhältlich ist, mit einem bestimmten Farbwert $FW(F_x)$ dieser Farbwert mit auf einen gewünschten Zielfarbwert $FW(Z_x)$ angepasst werden kann. Der Zielfarbwert ergibt sich insbesondere durch Vergleich mit der bestehenden Zahnsituation eines Patienten und berücksichtigt die individuelle Zahnfärbung des Patienten. Auf die Ausgangsrestauration wird daher eine farbige Glasur aufgetragen, deren Farbwert $FW(G_x)$ der Differenz zwischen dem Farbwert $FW(F_x)$ der Ausgangsrestauration und dem gewünschten Zielfarbwert $FW(Z_x)$ entspricht.

Figur 2 zeigt eine typische Farbskala zur Bestimmung und Auswahl von dentalen Formkörpern (VITA Toothguide 3D-MASTER®)

Ausgehend von einer keramischen Restauration mit einem Grundfarbwert, beispielsweise $FW(F_1)$ ermöglicht die vorliegende Erfindung eine einfache und effiziente Farbanpassung auf einen Zielfarbwert $FW(Z_1)$ durch Aufbringen einer Glasur, deren Farbwert $FW(G_1)$ der Differenz der Farbwerte $FW(F_1)$ und $FW(Z_1)$ entspricht. Je nach Anforderung kann dies einstufig oder mehrstufig erfolgen. Die Auswahl geeigneter Glasuren erfolgt anhand einer anerkannten Farbwerteskala, unter Berücksichtigung des Farbtons und der Farbintensität.

Als Ausgangformkörper dient eine Krone mit einem Farbwert FW $(F_2)$, der der Bezeichnung 3M2 aus der Farbskala VITA SYSTEM 3D-MASTER® entspricht. Dieser Ausgangsformkörper soll farblich so angepasst werden, dass er dem Zielwert FW $(Z_5)$ entspricht (s. Figur 1), welcher in diesem Beispiel der Zahnfarbe 3M3 aus der Farbskala VITA SYSTEM 3D-MASTER® entspricht. Um dies zu erreichen wird eine farbige Glasur ausgewählt und aufgebracht, deren Farbwert bei einer Auftragsdicke von ca. 30 $\mu$m der Differenz zwischen dem Ausgangsfarbwert und dem Zielfarbwert entspricht. Tabelle 1 verdeutlicht das erfindungsgemäße Vorgehen:

Tabelle 1

| Produkt | Farbwert | Transmission [%] | L* | a* | b* | $\Delta$E* | $\Delta$L | $\Delta$a | $\Delta$b |
|---|---|---|---|---|---|---|---|---|---|
| Ausgangskrone | FW(2) | | 76,30 | 3,36 | 20,24 | | | | |
| farbige Glasur * | FW(G5) | 43,31 | 74,43 | 3,33 | 23,76 | | | | |
| Zielfarbort | FW(Z5, soll) | | 76,40 | 3,80 | 22,50 | 2,27 | -0,10 | -0,17 | -2,26 |
| Krone+ Glasur | FW(Z5, ist) | | 76,48 | 3,70 | 21,88 | 0,63 | 0,08 | -0,10 | -0,62 |
| * Farbe wurde in Transmission gemessen, da es sich um eine transluzente Glasur handelt. | | | | | | | | | |

Bei den Farbwerten der Kronen handelt es aufgrund der höheren Opazität um Messungen in Reflexion.

Wie den Werten in Tabelle 1 zu entnehmen ist, wird durch das Aufbringen der farbigen Glasur auf die Ausgangskrone eine gute Übereinstimmung mit dem gewünschten Zielfarbwert erreicht.

**Patentansprüche**

1. Verfahren zur Herstellung einer dentalen Restauration, umfassend die folgenden Schritte:

    a) Bereitstellen eines Formkörpers für dentale Restaurationen, wobei der Formkörper einen Grundfarbwert FW(F) aufweist und wobei der Grundfarbwert FW(F) des Formkörpers bezüglich eines Zielfarbwerts FW(Z) der dentalen Restauration einen maximalen Differenzwert AE von 5 aufweist;
b) Aufbringen einer Glasur auf den Formkörper, wobei die Glasur einen definierten Farbwert FW(Gx) aufweist;
c) Wärmebehandeln des glasierten Formkörpers unter Erhalt der dentalen Restauration,

**dadurch gekennzeichnet, dass** der definierte Farbwert FW(Gx) der Glasur der Differenz AE zwischen den Farbwerten FW(Z) und FW(F) entspricht, wobei es sich bei den Farbwerten um CIEL*a*b*-Werte handelt und die und die Glasur in einer Dicke von 10 bis 50 pm, vorzugsweise 20 bis 40 pm auf den Formkörper aufgetragen wird.

2. Verfahren gemäß Anspruch 1, **dadurch gekennzeichnet, dass** die Glasur mit definiertem Farbwert durch Auswahl aus einer Reihe von Glasuren mit definierten Farbwerten FW(G) bereitgestellt wird.

3. Verfahren gemäß wenigstens einem der Ansprüche 1 oder 2, **dadurch gekennzeichnet, dass** der Formkörper durch Auswahl aus einem Set von Formkörpern mit definierten Grundfarbwerten FW(F) durch Vergleich mit den zur Restauration benachbarten Zähnen im Mund des Patienten mit Hilfe einer Farbskala bereitgestellt wird.

4. Verfahren gemäß wenigstens einem der vorangehenden Ansprüche, **dadurch gekennzeichnet, dass** es sich bei den Grundfarbwerten um solche handelt, die den Farbwerten in den standardisierten Farbskalen zur Zahnfarbenbestimmung entsprechen.

5. Verfahren gemäß wenigstens einem der vorangehenden Ansprüche, **dadurch gekennzeichnet, dass** die Bestimmung von AE und/oder die Auswahl des Formkörpers computergestützt erfolgt.

6. Verfahren gemäß wenigstens einem der vorangehenden Ansprüche, **dadurch gekennzeichnet, , dass** eine Glasur verwendet wird, die

   i) eine flüssige Anmischphase; und
   ii) eine feste Phase umfassend färbende Substanzen

   aufweist,
   wobei der Anteil an flüssiger Anmischphase in der Glasur mindestens 60 Gew.-% beträgt, bezogen auf das Gesamtgewicht der Glasur, und wobei die Glasur einen definierten Farbwert FW(Gx) aufweist.

7. Verfahren gemäß wenigstens einem der vorangehenden Ansprüche,, **dadurch gekennzeichnet, dass** die flüssige Anmischphase i) der Glasur ausgewählt ist aus der Gruppe bestehend aus Wasser, einem oder mehreren Alkoholen und Mischungen hiervon, wobei der Alkohol vorzugsweise ausgewählt ist aus der Gruppe der 1-, 2- und 3-wertigen Alkohole sowie Mischungen hiervon, insbesondere aus der Gruppe bestehend aus Glycerin, 1,3-Butandiol, 1,2-Propandiol, Ethanol und Mischungen hiervon.

8. Verfahren gemäß wenigstens einem der vorangehenden Ansprüche, , **dadurch gekennzeichnet, dass** die flüssige Anmischphase i) der Glasur weiterhin einen oder mehrere Komplexbildner, vorzugsweise ausgewählt aus der Gruppe bestehend aus Ethylendiamintetraessigsäure und Citronensäure, aufweist.

9. Verfahren gemäß wenigstens einem der vorangehenden Ansprüche, , **dadurch gekennzeichnet, dass** die Glasur in Form eines Kits vorliegt, das Kit umfassend einen ersten Behälter umfassend die flüssige Anmischphase und einen zweiten Behälter umfassend die feste Phase mit färbenden Substanzen.

10. Kit zur Durchführung eines Verfahrens zur Herstellung einer dentalen Restauration gemäß wenigstens einem der vorangehenden Ansprüche umfassend

   a) ein Sortiment an Glasuren für Formkörper für dentale Restaurationen, wobei jede Glasur einen definierten Farbwert F(Gx) aufweist, wobei die Farbwerte F(Gx) den Differenzen AE zwischen den Farbmustern einer standardisierten Farbskala zur Zahnfarbenbestimmung entsprechen und wobei AE nicht größer als 5 ist; und
   b) einen Farbführer zur Zuordnung eines ermittelten AE-Werts zu der entsprechenden Glasur.

11. Kit gemäß Anspruch 10, **dadurch gekennzeichnet, dass** das Sortiment 2 oder mehr, vorzugsweise 3 oder mehr, besonders bevorzugt 4 oder mehr Glasuren und/oder nicht mehr als 10, vorzugsweise nicht mehr als 8, besonders bevorzugt nicht mehr als 6 Glasuren umfasst.

12. Kit gemäß wenigstens einem der Ansprüche 10 oder 11, **dadurch gekennzeichnet, dass** das Kit weiterhin einen Applikator zum Aufbringen der Glasur auf einen Formkörper für dentale Restaurationen umfasst.

13. Kit gemäß wenigstens einem der Ansprüche 10 bis 12, **dadurch gekennzeichnet, dass** es sich bei den Glasuren um Glasuren gemäß wenigstens einem der Ansprüche 6 bis 9 handelt.

14. Verwendung einer Glasur mit einem definierten Farbwert FW(Gx) in einem Verfahren zur Herstellung dentalen Restauration gemäß einem der vorangehenden Verfahrensansprüche, wobei der Farbwert FW(Gx) der Differenz AE zwischen den Farbmustern einer standardisierten Farbskala zur Zahnfarbenbestimmung entspricht und wobei AE nicht größer als 5 ist.

**Claims**

1. A process for producing a dental restoration, comprising the following steps:

    a) providing a molded part for dental restorations, wherein said molded part has a basic color value FW(F), and wherein said color value FW(F) of the molded part has a maximum difference value $\Delta E$ of 5 from a target color value FW(Z) of the dental restoration;
    b) applying a glaze to the molded part, wherein said glaze has a defined color value FW(Gx);
    c) heat-treating said glazed molded part to obtain the dental restoration,

    **characterized in that** the defined color value $FW(G_x)$ of the glaze corresponds to the difference AE between the color values FW(Z) and FW(F), wherein said color values being CIEL*a*b* values and the glaze is applied to the molded part in a thickness of from 10 to 50 pm, preferably from 20 to 40 pm.

2. The process according to claim 1, **characterized in that** the glaze is provided with a defined color value by selecting from a number of glazes having defined color values FW(G).

3. The process according to at least one of claims 1 or 2, **characterized in that** the molded part is provided by selecting from a set of molded parts each having defined basic color values FW(F) by comparing with the teeth neighboring the restoration in the patient's mouth by using a color scale.

4. The process according to at least one of the preceding claims, **characterized in that** the basic color values are those that correspond to the color values in the standardized color scales for tooth color determination.

5. The process according to at least one of the preceding claims, **characterized in that** the determination of the AE and/or the selection of the molded part is effected with computer assistance.

6. The process according to at least one of the preceding claims, **characterized in that** a glaze is used that

    i) has a liquid mixing phase and
    ii) a solid phase including coloring substances,

    wherein the proportion of the liquid mixing phase in the glaze is at least 60% by weight, based on the total weight of the glaze, and wherein said glaze has a defined color value FW(Gx).

7. The process according to at least one of the preceding claims, **characterized in that** the liquid mixing phase i) of the glaze is selected from the group consisting of water, one or more alcohols, and mixtures thereof, in which said alcohol is preferably selected from the group of monohydric, dihydric and trihydric alcohols, and mixtures thereof, especially from the group consisting of glycerol, 1,3-butanediol, 1,2-propanediol, ethanol, and mixtures thereof.

8. The process according to at least one of the preceding claims, **characterized in that** the liquid mixing phase i) of the glaze further includes one or more complexing agents, preferably selected from the group consisting of ethylenediaminetetra-acetic acid, and citric acid.

9. The process according to at least one of the preceding claims, **characterized in that** said glaze is in the form of a kit, said kit comprising a first container including the liquid mixing phase, and a second container including the solid phase with coloring substances.

10. A kit for performing a process for producing a dental restoration according to at least one of the preceding claims, including

   a) an assortment of glazes for molded parts for dental restorations, wherein each glaze has a defined color value $F(G_x)$, in which the color values $F(G_x)$ correspond to the differences AE between the color samples of a standardized color scale for tooth color determination, and in which AE is not higher than 5; and
   b) a color guide for assigning an established AE value to the corresponding glaze.

11. The kit according to claim 10, **characterized in that** said assortment includes 2 or more, preferably 3 oder more, more preferably 4 or more, glazes and/or not more than 10, preferably not more than 8, more preferably not more than 6, glazes.

12. The kit according to at least one of claims 10 or 11, **characterized in that** the kit further includes an applicator for applying the glaze to a molded part for dental restorations.

13. The kit according to at least one of claims 10 to 12, **characterized in that** said glazes are glazes according to at least one of claims 6 to 9.

14. Use of a glaze with a defined color value $FW(G_x)$ in a process for producing dental restorations according to any of the preceding process claims, in which the color value $FW(G_X)$ corresponds to the difference AE between the color samples of a standardized color scale for tooth color determination, and in which AE is not higher than 5.

**Revendications**

1. Procédé pour préparer une restauration dentaire, comprenant

   les étapes consistant à :

   a) procurer un corps de moule pour restaurations dentaires, dans lequel le corps de moule a une valeur de couleur de base FW(F) et dans lequel la valeur de couleur de base FW(F) du corps de moule a une valeur de différence maximale AE de 5 par rapport à une valeur de couleur cible FW(Z) de la restauration dentaire ;
   b) appliquer un émail sur le corps moulé, cet émail ayant une valeur de couleur définie FW(Gx) ;
   c) soumettre le corps moulé émaillé à un traitement thermique pour obtenier la restauration dentaire,

   **caractérisé en ce que** la valeur de couleur définie FW(Gx) de l'émail correspond à la différence AE entre les valeurs de couleur FW(Z) et FW(F), où les valeurs de couleur sont des valeurs CIEL*a*b* et l'émail est appliqué sur le corps moulé sur une épaisseur de 10 à 50 pm, de préférence de 20 à 40 pm.

2. Procédé selon la revendication 1, **caractérisé en ce que** l'émail avec la valeur de couleur définie est procuré en sélectionnant parmi une gamme d'émaux aux valeurs de couleur définies FW(G).

3. Procédé selon au moins l'une des revendications 1 ou 2, **caractérisé en ce que** le corps de moule est fourni en sélectionnant parmi un ensemble de corps de moule avec des valeurs de couleur de base définies FW(F) en les comparant aux dents adjacentes à la restauration dans la bouche du patient à l'aide d'une échelle de couleurs.

4. Procédé selon au moins l'une des revendications précédentes, **caractérisé en ce que** les valeurs de couleur de base sont celles qui correspondent aux valeurs de couleur des échelles de couleurs normalisées pour la détermination de la couleur des dents.

5. Procédé selon au moins l'une des revendications précédentes, **caractérisé en ce que** la détermination de l'AE et/ou la sélection du corps de moule sont assistées par ordinateur.

6. Procédé selon au moins l'une des revendications précédentes, **caractérisé en ce qu'**un émail est utilisé qui présente

   i) une phase de mélange liquide ; et

ii) une phase solide comprenant des substances colorantes,

dans lequel la proportion de phase de mélange liquide dans l'émail est d'au moins 60 % en poids, basée sur le poids total de l'émail, et dans lequel l'émail a une valeur de couleur définie FW(Gx).

7. Procédé selon au moins l'une des revendications précédentes, **caractérisé en ce que** la phase de mélange liquide i) de l'émail est choisie dans le groupe constitué d'eau, d'un ou plusieurs alcools et de leurs mélanges, dans lequel l'alcool est de préférence choisi dans le groupe constitué d'alcools 1-, 2- et 3-hydriques et de leurs mélanges, en particulier dans le groupe constitué de glycérol, de 1,3-butanediol, de 1,2-propanediol, d'éthanol et de leurs mélanges.

8. Procédé selon au moins l'une des revendications précédentes, **caractérisé en ce que** la phase de mélange liquide i) de l'émail comprend en outre un ou plusieurs agents complexants, de préférence choisis parmi l'acide éthylènediaminetétraacétique et l'acide citrique.

9. Procédé selon au moins l'une des revendications précédentes, **caractérisé en ce que** l'émail est fourni sous forme de kit, celui-ci comprenant un premier récipient contenant la phase liquide de mélange et un second récipient contenant la phase solide avec des substances colorantes.

10. Kit for effectuer un procédé pour préparer une restauration dentaire selon au moins l'une des revendications précédentes, comprenant

a) une gamme d'émaux pour restaurations dentaires, chaque émail ayant une valeur de couleur définie F(Gx), les valeurs de couleur F(Gx) correspondant aux différences AE entre les échantillons de couleur d'une échelle de couleur dentaire standardisée et dans laquelle AE n'est pas supérieur à 5 ; et
b) un guide de couleurs pour attribuer une valeur AE déterminée à l'émail correspondant.

11. Kit selon la revendication 10, **caractérisé en ce que** la gamme comprend 2 ou plus, de préférence 3 ou plus, et plus particulièrement 4 ou plus d'émaux et/ou pas plus de 10, de préférence pas plus de 8, et plus particulièrement pas plus de 6 émaux.

12. Kit selon au moins l'une des revendications 10 ou 11, **caractérisé en ce que** le kit comprend en outre un applicateur pour appliquer l'émail sur un corps de moule destiné aux restaurations dentaires.

13. Kit selon au moins l'une des revendications 10 à 12, **caractérisé en ce que** les émaux sont des émaux selon au moins l'une des revendications 6 à 9.

14. Utilisation d'un émail avec une valeur de couleur définie FW(Gx) dans un procédé pour préparer des restaurations dentaires selon l'une des revendications de procédé précédentes, où la valeur de couleur FW(Gx) correspond à la différence AE entre les échantillons de couleur d'une échelle de couleur standardisée pour déterminer la couleur des dents et où AE n'est pas supérieur à 5.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69319291
- EP 3643272 A
- WO 2020156855 A
- EP 3650821 A
- WO 2020154450 A
- DE 102015204109
- US 20210322283 A
- US 4828117 A
- CN 108784875
- WO 2020185086 A
- US 5906490 A

**In der Beschreibung aufgeführte Nicht-Patentliteratur**

- **A. BALTZER** ; **V. KAUFMANN-JINOIAN**. Die Bestimmung der Zahnfarben. *QZ Quintessenz Zahntechnik*, July 2004, vol. 30